(19) Europäisches Patentamt
European Patent Office
Office européen des brevets

(11) Veröffentlichungsnummer: **0 001 996**
**B1**

(12) # EUROPÄISCHE PATENTSCHRIFT

(21) Anmeldenummer: **78101247.1**

(22) Anmeldetag: **28.10.78**

(51) Int. Cl.³: **C 07 F 9/40**, C 08 K 5/53, C 08 L 75/04

(54) **Verfahren zur Herstellung von N,N-Bis-(2-hydroxyalkyl)-aminomethanphosphonsäure-dimethylester und ihre Verwendung als flammhemmende Mittel für Kunststoffe**

(30) Priorität: **11.11.77 DE 2750555**

(43) Veröffentlichungstag der Anmeldung:
**30.05.79 Patentblatt 79/11**

(45) Bekanntmachung des Hinweises auf die Patenterteilung:
**23.07.80 Patentblatt 80/15**

(84) Benannte Vertragsstaaten:
**BE CH DE FR GB NL SE**

(56) Entgegenhaltungen:
**DE - A - 2 339 393**
**DE - A - 2 506 442**
**US - A - 3 076 010**
**US - A - 3 297 796**

(73) Patentinhaber: **Bayer AG**
**Zentralbereich Patente, Marken und Lizenzen**
**D-5090 Leverkusen 1, Bayerwerk (DE)**

(72) Erfinder: **Mitschke, Karl-Heinz, Dr.**
**Am Berg 22**
**D-5068 Odenthal (DE)**
**Kapps, Manfred, Dr.**
**Hoppersheiderweg 55**
**D-5060 Bergisch-Gladbach 2 (DE)**

**EP 0 001 996 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von N,N-Bis-(2-hydroxyalkyl)-aminomethanphosphonsäure - dimethylester und ihre Verwendung als flammhemmende Mittel für Kunststoffe

Die vorliegende Erfindung betrifft ein Verfahren zur Herstellung von N,N-Bis-(2-hydroxyalkyl) - aminomethanphosphonsäure - dimethylester der allgemeinen Formel

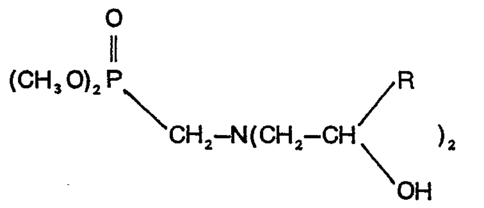

(I)

wobei

R gleich oder verschieden für Wasserstoff oder insbesondere für einen niederen, geradlinigen oder verzweigten Alkylrest oder Halogenalkylrest mit 1 bis 6 C-Atomen steht.

Verbindungen der obigen Formel, die hauptsächlich als flammhemmende Zusätze zu Kunststoffen, insbesondere für Polyurethane, eingesetzt werden, sind im Zusammenhang mit höheren Homologen dieser Verbindungsklasse zwar erwähnt worden, die Herstellung nach den bekannten Methoden führt jedoch zu keinem brauchbaren Ergebnis (vgl. z.B. US—PS 3 076 010 und US—PS 3 297 796). Bisher standen nur z.B. der Diäthylester der Bis-(hydroxyäthyl) - aminomethanphosphonsäure, der durch Umsetzung von Diäthanolamin mit wäßrigem Formalin und Diäthylphosphit hergestellt wird, zur Verfügung (vgl. z.B. DT—AS 1 143 022). Die entsprechenden Dimethylester, die natürlich von großem Interesse wären, konnten bislang noch nicht in befriedigender Weise hergestellt werden.

Auch das Verfahren gemäß der DE—OS 25 06 442, bei dem Oxazolidine mit Dialkylphosphiten ($C_2$ bis $C_{10}$ im alkylrest) in Gegenwart von sauren Ionenaustauschern umgesetzt werden, liefert nur unbefriedigende Ausbeuten an Dimethylester.

Bei der Herstellung der Verbindungen obiger Formel treten somit nach den bekannten Verfahren in starkem Maße Nebenreaktionen auf. U.a. wird auf die alkylierende Wirkung, besonders der niederen Alkylphosphite, aber auch der Dimethylphosphonate gegenüber Aminen bereits in der Literatur hingewiesen (Houben-Weyl), Methoden der organischen Chemie, Bd. 12/2, S. 10 und Bd. 12/1, S. 411).

Ähnliches tritt bei der Umsetzung von Alkanolaminen und Formalin mit Dimethylphosphit auf. Zusätzlich bewirkt das bei der Umsetzung von Dialkanolamin und Formalin vorliegende Wasser die Hydrolyse der Phosphit-Komponente, die dann nicht mehr in den gewünschten Reaktionsablauf eingreifen kann. Eine weitere Nebenreaktion ist die Umesterung der am Phosphor gebundenen Methoxy-Gruppe mit den endständigen Hydroxyl-Gruppen im Alkanolamin. Die Umesterung ist bekanntlich bei Methylgruppen enthaltenden Phosphiten mehr begünstigt als bei höheren Homologen.

Gegenstand der vorliegenden Erfindung ist ein Verfahren zur Herstellung von N,N-Bis-(2-hydroxyalkyl) - aminomethan - phosphonsäure - dimethylester der Formel

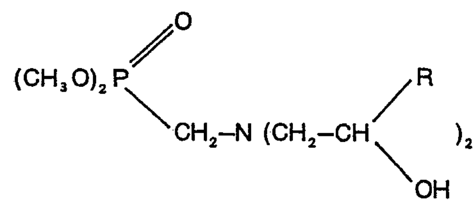

(I)

wobei

für R gleich oder verschieden für Wasserstoff oder insbesondere für einen niederern, geradkettigen oder verzweigten Alkylrest oder Halogenalkylrest mit 1—6 C-Atomen steht, durch Umsetzung von Oxazolidinen der allgemeinen Formel

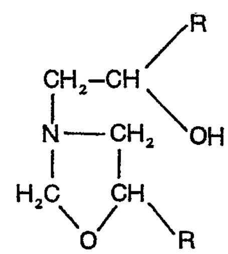

(II)

wobei

R die gleiche Bedeutung wie in (I) besitzt.

mit Dimethylphosphit, welches dadurch gekennzeichnet ist, daß die Umsetzung in Gegenwart H-acider Verbindungen durchgeführt wird.

Überraschenderweise hat sich herausgestellt, daß N,N-Bis-(2-hydroxyalkyl)-aminomethanphosphonsäure-dimethylester der Formel (I) in kurzer Zeit und mit bislang unerreichbaren Ausbeuten hergestellt werden können, wenn erfindungsgemäß in Gegenwart von H-aciden Verbindungen gearbeitet wird.

Das erfindungsgemäße Verfahren kann sowohl kontinuierlich — im allgemeinen mehrstufig in einer Reaktionskaskade — als auch diskontinuierlich durchgeführt werden.

Beim diskontinuierlichen Verfahren wird bevorzugt das Dimethylphosphit zusammen mit der H-aciden Verbindung vorgelegt und die Verbindung (II) zudosiert. Es ist aber auch möglich, das Dimethylphosphit allein vorzulegen und die Verbindung (II) zusammen mit der H-aciden Verbindung nachzudosieren. In Abhängigkeit von der Größe des Restes R in Verbindung (II) liegen die Reaktionstemperaturen zwischen 0 und 100°C, bevorzugt zwischen 20 und 60°C, die Reaktionszeiten belaufen sich auf 0,5—6 Stunden, bevorzugt zwischen 1 und 4 Stunden. Die Reaktion wird vorzugsweise lösungsmittelfrei durchgeführt, es ist aber auch möglich, geeignete Lösungsmittel wie z.B. Benzol, Toluol oder Petroläther zu verwenden.

Nach der Zudosierung der Komponenten wird bevorzugt nachgeheizt. Der Ablauf der Reaktion wird mit Hilfe der NMR-Spektroskopie verfolgt. Die Abnahme des P-H-Signals des Dimethylphosphits und der charakteristischen Signale der Verbindung (II) sind ein Maß für den Umsetzungsgrad der Reaktion. Weiterhin ist NMR-spektroskopisch auch der Grad der Nebenreaktionen gut zu erkennen (Umesterung, Alkylierung, eventuell Hydrolyse). Die resultierenden Verbindungen, besonders der N,N - Bis - (2 - hydroxypropyl) - aminomethanphosphonsäure - dimethylester, sind farblose bis schwach gelb gefärbte, klare Flüssigkeiten, die gut und rückstandslos in üblichen Lösungsmitteln löslich sind.

Als Katalysatoren werden erfindungsgemäß H-acide Verbindungen eingesetzt. Dafür kommen anorganische und organische mono- und polyfunktionelle Säuren in Frage. Zum Beispiel eignen sich Schwefelsäure, Salzsäure, Salpetersäure, Phosphorsäure, Mono- und Polycarbonsäuren wie z.B. Essigsäure, Chloressigsäure, Benzoesäure, Salicylsäure, Oxalsäure, Malonsäure, Phthalsäure, Sulfonsäuren, wie z.B. Alkyl- und Arylsulfonsäuren, Phosphon- und Phosphinsäuren, wie z.B. Alkyl- und Arylphosphon- und phosphinsäuren und Phenole; weiterhin deren Derivate wie beispielsweise Partialester- und Amide der Phosphorsäure, Phosphonsäure und Polycarbonsäuren.

Es können auch Kombinationen von H-aciden Verbindungen untereinander oder auch Gemische von Säuren mit sauren Ionenaustauschermaterialien eingesetzt werden.

Besonders wirksam sind H-acide Verbindungen mit einem $pK_s$-Wert von etwa -3 bis 10, vorzugsweise von 1—6 (die $pK_s$-Werte beziehen sich auf Werte in wäßrigem Medium). Besonders bevorzugt werden phosphorhaltige Verbindungen eingesetzt.

Bevorzugt werden die H-aciden Verbindungen in wasserfreiem Zustand verwendet. Die Mengen belaufen sich je nach Molekulargewicht und H-Funktionalität auf 0,1 bis 25 Mol-%, bezogen auf das Endprodukt, bevorzugt jedoch 2 bis 15 Mol-%. Höhere Anteile sind prinzipiell auch möglich, aufgrund der dabei resultierenden hohen Erniedrigung des pH-Wertes der Reaktionsmischung aber nicht sinnvoll.

Die verwendeten Oxazolidine der Formel (II) werden aus Bis - (2 - hydroxyalkyl) - aminen und Formaldehyd hergestellt, wobei bevorzugt Formaldehyd vorgelegt und das Dialkanolamin bei etwa 40—50°C zudosiert wird. Als Alkanolamine können z.B. Diäthanolamin, Diisopropanolamin oder andere entsprechende Amine eingesetzt werden. Vor dem Einsatz werden die Oxazolidine nach Möglichkeit wasserfrei gemacht oder destilliert.

Nach dem erfindungsgemäßen Verfahren können die Methylhomologen, Grundkörper der Verbindungsklasse gemäß Formel (I), erstmals in hoher Reinheit und Ausbeute synthetisiert werden. Damit lassen sich auf einfache Weise auch Verbindungen herstellen, die endständige sek. Hydroxylgruppen aufweisen, und damit beim Verschäumen mit Polyolen und Diisocyanaten gegenüber Verbindungen, hergestellt nach den bekannten Verfahren, verbesserte und gleichbleibende Qualitäten resultieren. Dieses Ergebnis ist auf das Fehlen von Nebenprodukten, die durch Hydrolyse, Umesterung oder Alkylierung entstehen und auf nicht umgesetzte Ausgangsmaterialien zurückzuführen. Diese Befunde werden NMR-spektroskopisch belegt.

Die erfindungsgemäß zugesetzten H-aciden Stoffe können im allgemeinen in den Reaktionsprodukten verbleiben und werden so gemeinsam mit dem Ester der Formel I, z.B. als Flammschutzmittel eingesetzt. Es ist aber auch möglich, die H-aciden Anteile z.B. in ihre entsprechenden Alkalisalze zu überführen und sie dann extraktiv zu entfernen. Auf diese Weise lassen sich die Ester isolieren.

Anhand der folgenden Beispiele soll das erfindungsgemäße Verfahren noch näher erläutert werden (%-Angaben sind, soweit nicht anders vermerkt ist, Gew.-%).

*Herstellung der Oxazolidine gemäß Formel II*

a) 29,5 kg (363 Mol, als 37%ige wäßrige Lösung) Formalin wurden in einem Kessel vorgelegt, auf ca. 40°C vorgewärmt und innerhalb von 3 Std. mit 47,9 kg (360 Mol) aufgeschmolzenem Diisopropanclamin (durch Zusatz von ca. 5% Wasser bleibt Diisopropanolamin bei Raumtemperatur flüssig) so schnell versetzt, daß die Innentemperatur bei 40—43°C, gegebenenfalls unter Kühlung, gehalten wurde. Nach dem Zudosieren wurde noch ca. 1 Std. bei 50°C nachgerührt. Das entstandene Oxazolidin der Formel II ($R = CH_3$) wurde innerhalb von 5,5 Std. bei 80°C, gegen Ende bei 60°C, im Dampfstrahlvakuum (ohne Zwischenschaltung einer Kolonne) entwässert. Es wurden 50,6 kg (96,5% der Theorie) Produkt mit einer Reinheit von 99,85% (GC) und einem Wassergehalt von ca. 0,1% erhalten.

b) Analog wurden aus 1230 g (15,15 Mol, als 37%ige wäßrige Lösung) Formalin und 1577 g (15 Mol) Diäthanolamin, 1670 g (95% der Theorie) des Oxazolidins der Formel II ($R = H$) mit einer Reinheit von 96,8% (GC) erhalten.

Beispiel 1

In einem Kessel wurden 33 kg (300 Mol) Dimethylphosphit und 2,91 kg Dibutylphosphat vermischt und auf 40—42°C erwärmt. Innerhalb von 2,5 Std. wurden 44,9 kg (309 Mol) Oxazolidin gemäß a) in diese Mischung eingetragen, wobei unter Kühlung die vorher genannte Temperatur aufrecht erhalten wurde. Gegen Ende der Zudosierung wurde die Temperatur langsam auf 50°C gesteigert und ca. 3 Stunden nachgerührt. Es wurden 80,4 kg

(99,5% der Theorie) einer hellgelben Flüssigkeit der Zusammensetzung

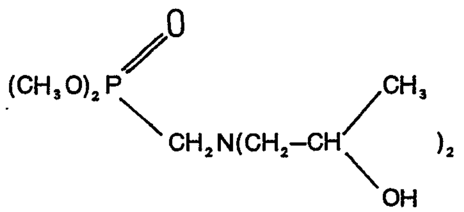

mit der Dichte 1,135 g/ml (25°C) und der Viskosität 754 cp (25°C) erhalten. Das Produkt enthielt noch 0,046 Mol (pro Mol der Dimethylverbindung),

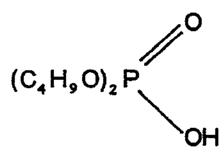

Beispiel 2 (Vergleich)
ohne H-acide Verbindungen

Zu 110 g (1 Mol) Dimethylphosphit wurden bei 40°C 145,2 g (1 Mol) Oxazolidin gemäß a) innerhalb von 30 Min. getropft und danach 3 Stunden bei 50°C nachgerührt.

Nach NMR-Messungen wurden noch ca. 13,5% Dimethylphosphit, ca. 4,5% Desmethylverbindung (durch Alkylierung entstanden), ca. 12% Oxazolidin und ca. 6% Methanol gefunden. Bei weiterem Erhitzen wurde die Umsetzung nicht vervollständigt, jedoch nahm die Nebenprodukt-Bildung und die Erhöhung der Viskosität zu.

Beispiel 3

Eine Mischung aus 110 g (1 Mol) Dimethylphosphit und 7,9 g Dibutylphosphat wurden innerhalb von 25 Min. unter Rühren und Kühlen mit 117 g (1 Mol) Oxazolidin gemäß b) versetzt. Anschließend wurde 3 Stunden bei 40°C nachgerührt. Man erhielt in quantitativer Ausbeute eine hellgelbe Flüssigkeit der Zusammensetzung

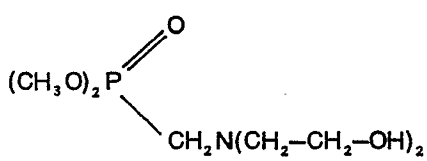

mit der Dichte 1,2259 g/ml (25°C) und der Viskosität 350 cP (25°C). (0,35 Pa's)

Beispiel 4 (Vergleich)
ohne H-acide Verbindungen

Zu 110 g (1 Mol) Dimethylphosphit wurden 117 g (1 Mol) Oxazolidin gemäß b) innerhalb von 25 Min. getropft und anschließend 3 Stunden bei 50°C nachgerührt.

Nach NMR-Messungen wurden noch ca. 5,5 Mol-% Dimethylphosphit, ca. 7,7 Mol-% Desmethylverbindung (durch Alkylierung entstanden), ca. 5 Mol-% Oxazolidin und ca. 7 Mol-% Methanol gefunden. Bei weiterem Erhitzen wurde die Umsetzung nicht vervollständigt, jedoch nahm die Viskosität unter Dunkelfärbung stark zu.

Beispiel 5

55 g (0,5 Mol) Dimethylphosphit und 3 g Chlormethanphosphonsäure wurden vermischt und bei 40°C unter Rühren und Kühlen mit 74,7 g (0,513 Mol) Oxazolidin gemäß a) versetzt. Anschließend wurde bei 50°C 3 Std. nachgerührt. Man erhielt in quantitativer Ausbeute eine hellgelbe Flüssigkeit der Zusammensetzung.

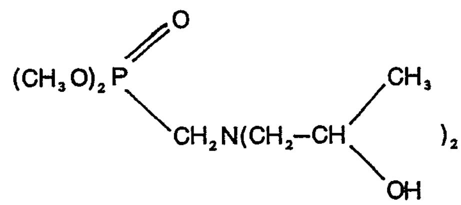

Das Produkt enthielt noch 0,044 Mol Chlormethanphosphonsäure (pro Mol der Dimethylverbindung). Die Chlormethanphosphonsäure kann durch Zugabe einer doppelt molaren Menge einer wäßrigen NaOH-Lösung in das entsprechende Na-Salz überführt werden. Das Produkt wird mit Toluol extrahiert, während das Salz in der wäßrigen Phase zurückbleibt.

In den vorher aufgeführten und den folgenden Beispielen wurde der Katalysator jeweils im Reaktionsgemisch belassen.

Beispiel 6

Entsprechend Beispiel 5 erhielt man aus 55 g (0,5 Mol) Dimethylphosphit, 2,8 g Tetramethylenphosphinsäure und 74,7 g (0,514 Mol) Oxazolidin gemäß a) in quantitativer Ausbeute eine hellgelbe Flüssigkeit der Zusammensetzung

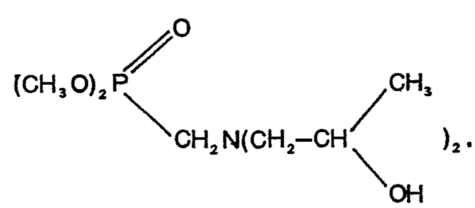

Beispiel 7

Entsprechend Beispiel 5 erhielt man aus 55 g (0,5 Mol) Dimethylphosphit, 2,4 g Vinylphosphonsäure und 74,7 g (0,514 Mol) Oxazolidin gemäß a) in quantitativer Ausbeute eine hellgelbe Flüssigkeit der Zusammensetzung

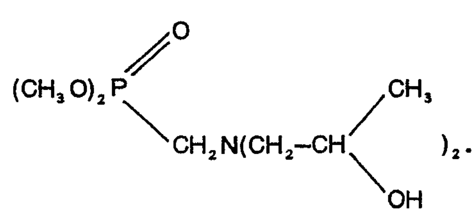

Beispiel 8

Entsprechend Beispiel 5 erhielt man aus 55 g (0,5 Mol) Dimethylphosphit, 4,9 g p-Toluolsulfonsäure und 74,7 g (0,514 Mol) Oxazolidin gemäß a) in quantitativer Ausbeute eine hellgelbe Flüssigkeit der Zusammensetzung

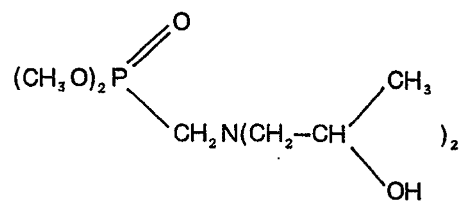

Beispiel 9

Entsprechend Beispiel 5 erhielt man aus 110 g (1 Mol) Dimethylphosphit, 8,8 g Phosphorsäure und 149,6 g (1,03 Mol) Oxazolidin gemäß a) in quantitativer Ausbeute eine hellgelbe Flüssigkeit der Zusammensetzung

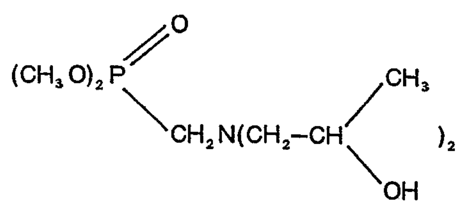

Beispiel 10

Entsprechend Beispiel 5 wurde zu 110 g (1 Mol) Dimethylphosphit eine Mischung aus 145,2 g (0,2 Mol Oxazolidin gemäß a) und 7,6 g Eisessig bei 40°C getropft und ca. 3 Stunden bei 45°C nachgerührt. Man erhielt in quantitativer Ausbeute eine hellgelbe Flüssigkeit der Zusammensetzung

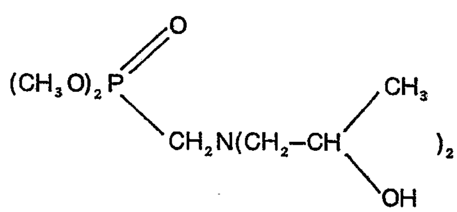

Beispiel 11

(Anwendung von Produkt gemäß Beispiel 1 als Flammschutzmittel in einem Polyurethan-Hartschaumstoff).

Verschäumungsrezeptur:

85 g eines durch Propoxylierung von Saccharose erhaltenen Polyätherpolyols (OH-Zahl 380, Viskosität bei 25°C 13000 mPa's)

15 g Produkt gemäß Beispiel 1

1,5 g eines Siliconstabilisators auf Basis eines mit seitenständigen Polyäthergruppen modifizierten Polysiloxans

0,5 g Wasser

2,0 g Dimethyl-cyclohexyl-amin

28,0 g Monofluortrichlormethan

115,0 g rohes 4,4'-Diisocyanato-diphenylmethan

Verschäumung:

Das Polyol wurde mit dem Produkt gemäß Beispiel 1, den Zusatzmitteln und dem Treibmittel durch Rühren vermischt und die durch den Mischvorgang verflüchtigte Treibmittelmenge ergänzt. Danach wurde das Isocyanat zugegeben und die Mischung intensiv verrührt. Alle Komponenten waren vor dem Vermischen auf 20°C ± 0,5°C temperiert worden. Das Reaktionsgemisch wurde sofort nach der Herstellung in eine Form aus Packpapier (Grundfläche 20 x 20 $cm^2$, Höhe 14 cm) gegossen. Während des Schäumvorganges wurden die Startzeit und die Abbindezeit gemessen; die Brennbarkeit wurde nach ASTM bestimmt.

Folgende Werte wurden erhalten:

Startzeit [s] 32

Abbindezeit [s] 145

Rohdichte [kg/$m^3$] 33,2

Druckfestigkeit [MPa] 0,24

(in Schäumrichtung)

Dimensionsstabilität

5 h + 100°C [Vol-%] keine Änderung

Brennbarkeit nach ASTM-D-1692

Abbrandstrecke [cm] 3,7 cm

Verlöschzeit [s] 35

**Patentansprüche**

1. Verfahren zur Herstellung von N,N - Bis - (2 - hydroxyalkyl - aminomethan - phosphonsäure - dimethylestern der allgemeinen Formel

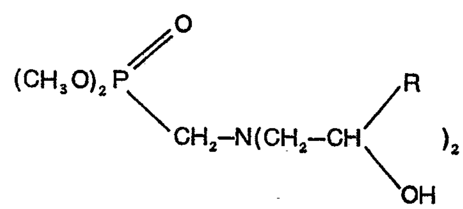 (I)

wobei

R gleich oder verschieden sein kann und für Wasserstoff oder insbesondere für einen geradlinigen oder verzweigten Alkyl- oder Halogenalkylrest mit 1—6 C-Atomen steht,

durch Umsetzung von Oxazolidinen der allgemeinen Formel

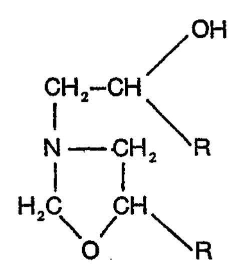 (II)

wobei

R die gleiche Bedeutung wie in (I) besitzt.

mit Dimethylphosphit, dadurch gekennzeichnet, daß die Umsetzung in Gegenwart H-acider Verbindungen durchgeführt wird.

2. Verfahren nach Anspruch 1, dadurch

gekennzeichnet, daß die Menge an H-acider Verbindung zwischen 0,1 und 25 Mol-%, bevorzugt zwischen 2 und 15 Mol% bezogen auf das Endprodukt beträgt.

3. Verfahren nach einem der Ansprüche 1 oder 2, dadurch gekennzeichnet, daß die Umsetzung zwischen 0 und 100°C, bevorzugt zwischen 20 und 60°C durchgesetzt werden.

4. Verwendung der nach den Ansprüchen 1 bis 3 hergestellten Verbindungen, gegebenenfalls im Gemisch mit den als Katalysator eingesetzten H-aciden Verbindungen, als flammhemmende Mittel für Kunststoffe, insbesondere für Polyurethane.

## Revendications

1. Procédé de production d'esters diméthyliques d'acides N,N-bis-(2-hydroxyalkyl)-aminométhane-phosphoniques formule générale:

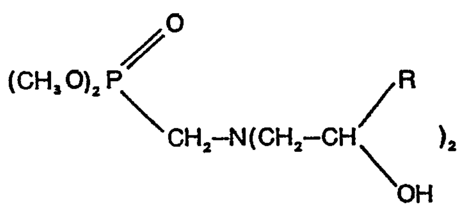 (I)

(dans laquelle les symboles R peuvent être égaux ou différents et peuvent représenter de l'hydrogène ou en particulier un reste alkyle ou halogénalkyle linéaire ou ramifié ayant 1 à 6 atomes de carbone) par réaction d'oxazolidines de formule générale:

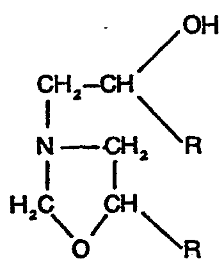 (II)

(dans laquelle R a la même définition que pour la formule (I)) avec le phosphite de diméthyle, caractérisé en ce que la réaction est conduite en présence de composés renfermant de l'hydrogène acide.

2. Procédé suivant la revendication 1, caractérisé en ce que la quantité de composé renfermant de l'hydrogène acide se situe entre 0,1 et 25 moles %, de préférence entre 2 et 15 moles %, par rapport au produit final.

3. Procédé suivant l'une des revendications 1 et 2, caractérisé en ce que la réaction est conduite entre 0 et 100°C et, de préférence, entre 20 et 60°C.

4. Utilisation de composés produits suivant l'une quelconque des revendications 1 à 3, éventuellement en mélange avec les composés renfermant de l'hydrogène acide utilisés comme catalyseur, comme retardateur de combustion pour matières plastiques, notamment pour polyuréthannes.

## Claims

1. A process for the preparation of N,N-bis-(2-hydroxylalkyl)-aminoethane phosphonic acid dimethyl esters of the general formula

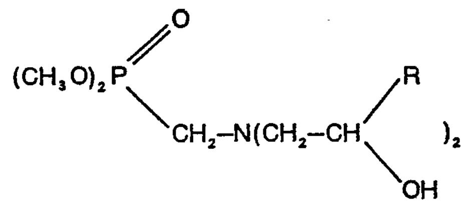 (I)

wherein

R may be the same or different and represents hydrogen or in particular a linear or branched alkyl or halogen alkyl radical containing 1 to 6 C atoms,

by reacting oxazolidines of the general formula

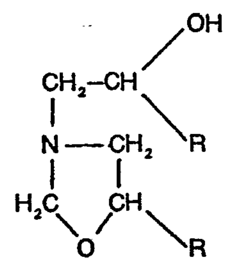 (II)

wherein

R has the same meaning as in (I) with dimethyl phosphite, characterised in that the reaction is carried out in the presence of H-acid compounds.

2. A process according to claim 1, characterised in that the quantity of H-acid compound is between 0.1 and 25 mole %, preferably between 2 and 15 mole %, based on the end product.

3. A process according to one of claims 1 or 2, characterised in that the reaction is carried out at between 0 and 100°C, preferably at between 20 and 60°C.

4. The use of the compounds produced in accordance with claims 1 to 3, optionally in admixture with the H-acid compounds used as catalyst, as flame retardants for plastics, particularly polyurethanes.